# EUROPEAN PATENT APPLICATION

(11) **EP 0 541 089 A2**
(43) Date of publication of application: **12.05.1993**
(21) Application number: 92118974.2
(22) Date of filing: 05.11.1992
(51) Int. Cl.: C12N 15/40, C12N 15/51, C12Q 1/70, G01N 33/569, A61K 39/29

(54) **cDNA of human hepatitis C virus, its clone and use thereof**

(30) Priority: 07.11.1991 JP 318679/91
(71) Applicant: SANWA KAGAKU KENKYUSHO CO., LTD., Higashi-ku Nagoya-shi Aichi-ken (JP)
(72) Inventor: Kurono, Masayasu, Higashi-ku, Nagoya (JP); Mitani, Takahiko, Higashi-ku, Nagoya (JP); Jomori, Takahito, Higashi-ku, Nagoya (JP); Hayashi, Yuji, Higashi-ku, Nagoya (JP); Suzuki, Eiji, Higashi-ku, Nagoya (JP); Sawai, Kiichi, Higashi-ku, Nagoya (JP)
(74) Representative: Wächtershäuser, Günter, Prof. Dr.

(57) **Abstract**

There are disclosed a cDNA of human hepatitis C virus gene, its clone and use thereof. The cDNA is prepared by constructing a cDNA library with an RNA, as a template, derived from a liver tissue or serum of a patient who was definitely diagnosed as infected with hepatitis C, carrying out a cDNA cloning with use of the library and probes encoding parts of nucleotide sequence which codes a known HCV virus gene to obtain clones reacting with the probes, carrying out further cloning with use of the reacted clones, determining a nucleotide sequence on each positive clone, and ligating peptides with the nucleotide sequence to establish as only one HCV clone. The HCV clone is constructed with about 9500 nucleotides having a sequence shown in the Sequence Listing.

## Description

The present invention relates to a cDNA of human hepatitis C virus (hereinafter referred to "HCV") gene, its clone and use thereof.

At the present time, hepatitis C occupies 90 to 95% among a family of blood transfusional hepatitises, and a positive remedy thereof has not yet been established. Therefore, there are strong demands for developing its diagnosing method, therapeutic drug and preventive vaccine.

In recent year, Chiron Corporation in U.S.A. has cloned cDNA derived from a liver tissue of a chimpanzee infected with human Non A Non B hepatitis (hereinafter referred to as "NANB"), and expressed with Escherichia coli which was transformed with a vector of λ gt11 with an insert of the DNA to find that a product thereof reacts with a serum sample derived from a patient infected with NANB. They have finally found that it is a single-stranded RNA virus having about 9000 nucleotides, which is similar to that of filavi virus, named the same as "HCV", and disclosed a part of its nucleotide sequence, namely about 7000 nucleotides among said about 9000 nucleotides [EP-0318216(A1)].

Then, several nucleotide sequences of HCV separated from Japanese patients infected with NANB have been reported by Okamoto et al. ["The Japan Journal of Experimental Medicine", 60, 167 - 177 (1990)], Kato et al. ["Proceedings of the National Academy of Sciences of the U.S.A.", 87, 9524 - 9528 (1990)], and Enomoto et al. ["Biochemical and Biophysical Research Communications", 170, 1021 - 1025 (1990)].

In case of comparing the sequences published in said literatures, there are recognized noticeable differences in the level of nucleotides and amino acids therein.

Said Chiron Corporation had developed a diagnosis kit which comprises an antigen comprising a polypeptide expressed with use of a part of said HCV gene in yeast and an antibody in serum of a patient infected with NANB reacting thereto, and which detects said antibody. They have reported that said kit could detect the antibody in the serum by the probability of about 70%, in epidemiological view point. The kit, however, has never possessed of an excellent detecting rate thereof.

As referred to hereinbefore, there is no accurate and sufficient information on the nucleotide sequence of HCV virus gene, it has never been established an exact and accurate diagnosing method, a therapeutic method, a treatment doing harmless of blood for transfusion and a development of preventive vaccine.

With respect to the structure of HCV gene, said Chiron Corporation in U.S.A. has isolated and analyzed the HCV from the liver tissue of chimpanzee infected with human NANB; Okamoto et al.,Kato et al. and Enomoto et al. have isolated and analyzed the HCV from the serum or plasma of Japanese individuals infected with NANB. The structure of HCV gene analyzed by Chiron Corporation, however, is derived from chimpanzee. Okamoto et al. and Enomoto et al. have merely reported to make clear a part of the sequences. While, though Kato et al. have reported the structure of HCV gene comprising about 9500 nucleotides which covers near full length thereof, they have extracted RNA from a pool serum of several patients infected with NANB to prepare the cDNA, but they have never obtained a single clone thereof. Therefore, the structure of HCVs among various single clones must accurately compare and investigate to check the variety thereof, and a result of such study have never been published hitherto.

In due consideration of the lack of information for the nucleotide sequence of HCV and the presence of a variety of HCV genes, more information for the structure of HCV is required to develop an exact and accurate diagnosis method, a therapeutic method, a treatment doing harmless of blood for transfusion and a preventive vaccine for Hepatitis C. Therefore, problems of the present invention lies in finding out of a new HCV strain different from known HCV strains.

The present inventors, in the first place, isolated and purified RNA from a liver tissue and serum derived from one patient who was definitely diagnosed as infected with NANB, and prepared a cDNA library by a method known per se using thus purified RNA as a template. On the basis of this cDNA library, cDNA cloning was practiced with probes, each of which comprises a part of the nucleotide sequence encoding the HCV gene disclosed by Kaneko et al.in Jap. Pat. No. Hei 3 (A.D. 1991) - 198777(A) to obtain some clones. These clones were finally ligated each other to prepare one HCV clone. The HCV clone has the nucleotide sequence and amino acid sequence shown in "Sequence Listing" given later.

The clone comprises all of nucleotide sequence of human HCV. Since the sequence for the clone is different from that for the HCV reported by Chiron Corporation in 22% or more at nucleotide level and 16% or more at amino acid level, the clone can be said as new cloned strain apparently different from conventional cloned HCV strains.

Antigenic substance due to HCV may be prepared in a large amount by selecting a suitable expression vector to prepare a recombinant vector wherein a double-stranded DNA fragment comprising the cDNA fragment according to the invention is inserted, transforming a host of Escherichia coli, yeast, or animal cell with the recombinant vector, and cultivating the transformant. The resulting product can be employed to measure an amount of anti-HCV antibody existing in the blood of patients to diagnose a degree of progress and recovery of hepatitis C or tumor caused by HCV and to diagnose carriers thereof. Additionally, said antigenic substance may be employed to be attenuated by the method of subculture and immunity, and thereby the way for development of vaccine can be opened. An animal may be immunized with the anitigenic substance to produce an antibody substance thereto, and then therapeutical drugs and treatment reagents doing harmless of blood for transfusion shall be provided with use of such an antibody substance. The antibody substance can be stably supplied as the therapeutic drugs, by preparing polyclone or monoclone thereof, with use of a method known per se.

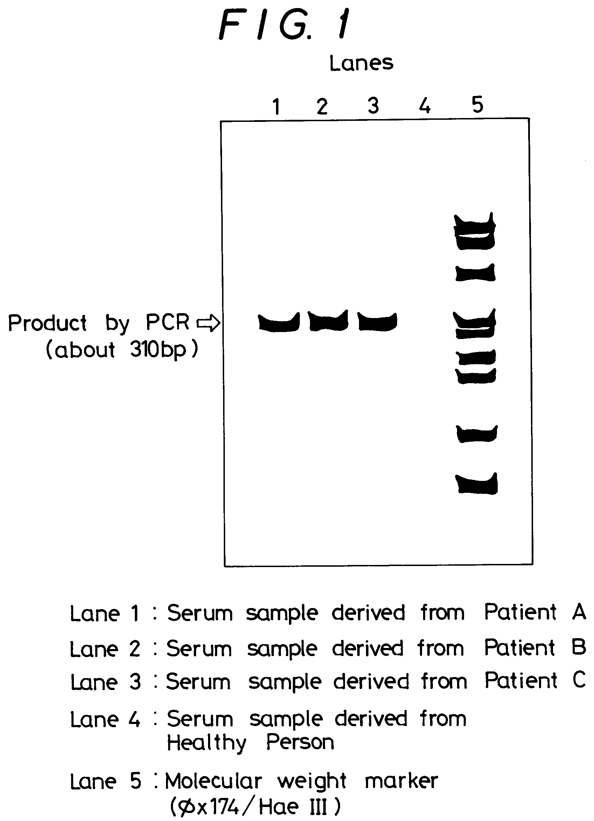

The invention will now be further explained in more detail and concretely with reference to Examples, which shall refer to a drawing, wherein
Fig.1 is patterns showing results of electrophoresis after a polymerase chain reaction using a cDNA which was synthesized by employing an RNA as a template, the RNA being derived from 3 patients who had been definitely diagnosed as infected with NANB and one healthy person, and using primers which are DNA fragments with nucleotide sequences of nucleotide numbers of 19th - 43rd as well as 304th - 328th in the Sequence Listing.

### Example 1

### (A) Isolation of RNA from a patient infected with NANB, purification thereof, and synthesis of cDNA

According to the method described in Jap. Pat. No. Hei 3 (A.D. 1991) - 30676(A), isolation of mRNA from a patient (only one person) infected with NANB and purification thereof was practiced. With use of resulting purified RNA, random hexamers as primers and a reverse transcriptase, a DNA fragment complementary to the RNA was synthesized. Then, a double-stranded DNA was synthesized by said cDNA and T4 DNA polymerase. Both ends of the resulting double-stranded DNA fragment was blunted, ligated EcoRI linker, digested with EcoRI and purified to afford the desired DNA fragment.

### (B) Preparation of cDNA library and its cloning

A phage vector of λ gt11 was selected as expression vector, cleaved with EcoRI, inserted a DNA fragment which was obtained by the step of said Item (A) and had EcoRI recognition sites on both ends thereof to construct a cDNA library according to the conventional in vitro packaging method. With use of the cDNA library, cloning was practiced by using, as a primer, a DNA which is a part of nucleotide sequence encoding HCV gene and disclosed by Kaneko et al. , said DNA comprising about 240 nucleotides [Jap. Pat. No. Hei 3 (A.D. 1991) - 198777(A)] to obtain some positive clones reacting with the probe. With use of the reacted clones as probes, cloning was further carried out to be finally selected 13 positive clones. Each of nucleotide sequences of the positive clones was determined by the dioxy method and same parts in these sequences were overlapped to establish as only one clone. As a result, it was clarified that this clone comprises about 9500 nucleotides. All of the nucleotide sequence of this clone and an amino acid sequence translated therefrom are shown in the "Sequence Listing" given later.

### Example 2

### (A) Genetic diagnosis of human hepatitis C using 5'-non-translational region of cloned DNA derived from HCV

From serum of patients assumed as infected with NANB, all RNAs were extracted by means of the method disclosed by Zirgwin et al. using guanidine thiocyanate ["Biochemistry", 18, 5294 - 5299 (1979)]. On this RNA, cDNA was prepared by using parts of the nucleotide sequence given in the Sequence Listing (sequence of 19th to 43rd nucleotides and complementary sequence of 304th to 328th nucleotides in the nucleotide number given in the Sequence Listing), as primers and incubating for 1 hour at 42°C in the presence of a reverse transcriptase to synthesize a cDNA. This cDNA was employed and subjected to PCR (Polymerase Chain Reaction) in accordance with the method disclosed by Kaneko et al. ["Journal of Clinical Microbiology", 27, 1930 - 1933 (1989)], and then subjected to an electrophoresis.

Results of the electrophoresis are shown in Fig.1, and an anticipated band with a length of about 310 base pairs was identified on each case of the patients. This fact means that a person who provides the subjected serum had been infected with hepatitis C, since said band was not detected, when a RNA derived from serum of healthy person was employed.

SEQUENCE LISTING

SEQ ID NO : 1
SEQUENCE TYPE : Nucleotide with corresponding protein
SEQUENCE LENGTH : 9405 Nucleotides

STRANDNESS : Single
TOPOLOGY : linear
MOLECULE TYPE : cDNA to genomic RNA

HYPOTHETICAL : No
ANTI-SENSE : No

ORIGINAL SOURCE
ORGANISM : Human
TISSUE TYPE : Liver and serum
PROPERTIES : Human C hepatitis virus gene

GGCGACACTC CACCATAGAT CACTCCCCTG TGAGGAACTG CTGTCTTCAC 50
GACGAAAGCG TCTAGCCATG GCGTTAGTAT GACTGTCGTG CAGCCTGCAG 100
GACCCCCCCT CCCGGGAGAG CCATAGTGGT CTGCGGAACC GGTGAGTACA 150
CCGGAATTGC CAGGACGACC GGGTCCTTTC TTGGATCACC CCGCTCAATG 200
CCTGGAGATT TGGGCATGCC CCCGCGAGAC TGCTAGCCGA GTAGTGTTGG 250
GTCGCGAAAG GCCTTGTGGT ACTGCCTGAT AGGGTGCTTG CGAGTGCCCC 300
GGGAGGTCTC GTAGACCGTG CATC ATG AGC ACA AAT CCT AAA CCT CAA 348
Met Ser Thr Asn Pro Lys Pro Gln
1               5
AGA AAA ACC AAA CGT AAC ACC AAC CGC CGC CCA CAG GAC GTC AAG TTC 396
Arg Lys Thr Lys Arg Asn Thr Asn Arg Arg Pro Gln Asp Val Lys Phe
    10                  15                  20
CCG GGC GGT GGC CAG ATC GTT GGT GGA GTT TAC CTG TTG CCG CGC AGG 444
Pro Gly Gly Gly Gln Ile Val Gly Gly Val Tyr Leu Leu Pro Arg Arg
25                  30                  35                  40
GGC CCC AGG TTG GGT GTG CGC GCG ACT AGG AAG ACT TCC GAG CGG TCG 492
Gly Pro Arg Leu Gly Val Arg Ala Thr Arg Lys Thr Ser Glu Arg Ser
                45                  50                  55

CAA CCT CGT GGA AGG CGA CAA CCT ATC CCC AAG GCT CGC CAG CCC GAG 540
Gln Pro Arg Gly Arg Arg Gln Pro Ile Pro Lys Ala Arg Gln Pro Glu
            60                  65                  70
GGC AGG GCC TGG GCT CAG CCC GGG TAC CCT TGG CCC CCC TAT GGC AAT 588
Gly Arg Ala Trp Ala Gln Pro Gly Tyr Pro Trp Pro Pro Tyr Gly Asn
        75                  80                  85
GAG GGT CTG GGG TGG GCA GGA TGG CTC CTG TCA CCC CGT GGC TCC CGG 636
Glu Gly Leu Gly Trp Ala Gly Trp Leu Leu Ser Pro Arg Gly Ser Arg
    90                  95                  100
CCT AGT TGG GGC CCC ACG GAC CCC CGG CGT AGG TCG CGT AAT TTG GGT 684
Pro Ser Trp Gly Pro Thr Asp Pro Arg Arg Arg Ser Arg Asn Leu Gly
105                 110                 115                 120
AAG GTC ATC GAT ACC CTC ACA TGC GGC TTC GCC GAC CTC ATG GGG TAC 732
Lys Val Ile Asp Thr Leu Thr Cys Gly Phe Ala Asp Leu Met Gly Tyr
                125                 130                 135
ATT CCG CTC GTC GGC GCC CCC CTA GGA GGC GCT GCC AGG GCC CTG GCG 780
Ile Pro Leu Val Gly Ala Pro Leu Gly Gly Ala Ala Arg Ala Leu Ala
            140                 145                 150
CAT GGC GTC CGG GTT CTG GAG GAC GGC GTG AAC TAC GCA ACA GGG AAT 828
His Gly Val Arg Val Leu Glu Asp Gly Val Asn Tyr Ala Thr Gly Asn
        155                 160                 165
CTG CCC GGT TGC TCT TTC TCT ATC TTC CTC TTG GCT CTG CTG TCT TGT 876
Leu Pro Gly Cys Ser Phe Ser Ile Phe Leu Leu Ala Leu Leu Ser Cys
    170                 175                 180
TTG ACC ATC CCA GCA TCC GCT TAT GAA GTG CGC AAC GTG TCC GGG TTG 924
Leu Thr Ile Pro Ala Ser Ala Tyr Glu Val Arg Asn Val Ser Gly Leu
185                 190                 195                 200
TAC CAT GTC ACG AAC GAC TGC TCC AAC TCA AGT ATT GTG TAT GAG GCA 972
Tyr His Val Thr Asn Asp Cys Ser Asn Ser Ser Ile Val Tyr Glu Ala
                205                 210                 215
GCG GAC TTG ATC ATG CAC ACC CCT GGG TGC GTG CCC TGC GTC CTG GAG 1020
Ala Asp Leu Ile Met His Thr Pro Gly Cys Val Pro Cys Val Leu Glu
            220                 225                 230
GGA AAC TCC TCC CGT TGC TGG GTA GCG CTC ACT CTC ACG CTC GCG GCC 1068
Gly Asn Ser Ser Arg Cys Trp Val Ala Leu Thr Leu Thr Leu Ala Ala
        235                 240                 245

AGG AAC AGC AGC ATC CCC ACT ACG ACA ATA CGA CGT CAC GTC GAC TTG 1116
Arg Asn Ser Ser Ile Pro Thr Thr Thr Ile Arg Arg His Val Asp Leu
    250                 255                 260
CTC GTT GGG GCG GCT GCT TTC TGC TCC GCT ATG TAC GTG GGG GAT CTC 1164
Leu Val Gly Ala Ala Ala Phe Cys Ser Ala Met Tyr Val Gly Asp Leu
265                 270                 275                 280
TGC GGA TCT GTT TTC CTC GTC TCC CAG CTA TTC ACC TTT TCA CCT CGC 1212
Cys Gly Ser Val Phe Leu Val Ser Gln Leu Phe Thr Phe Ser Pro Arg
                285                 290                 295
CGG TAT GAG ACG GTA CAA GAC TGC AAT TGC TCA CTC TAT TCC GGC CAC 1260
Arg Tyr Glu Thr Val Gln Asp Cys Asn Cys Ser Leu Tyr Ser Gly His
            300                 305                 310
GTA TCA GGT CAC CGC ATG GCC TGG GAT ATG ATG ATG AAC TGG TCG CCC 1308
Val Ser Gly His Arg Met Ala Trp Asp Met Met Met Asn Trp Ser Pro
        315                 320                 325
ACA ACA GCC CTA GTG GTA TCG CAG CTA CTC CGG ATC CCA CAA GCC GTC 1356
Thr Thr Ala Leu Val Val Ser Gln Leu Leu Arg Ile Pro Gln Ala Val
    330                 335                 340
GTG GAC ATG GTG GCG GGG GCC CAC TGG GGA GTC TTG GCA GGC CTC GCC 1404
Val Asp Met Val Ala Gly Ala His Trp Gly Val Leu Ala Gly Leu Ala
345                 350                 355                 360
TAC TAT TCC ATG GTG GGG AAC TGG GCT AAG GTC TTG ATT GTG ATG CTA 1452
Tyr Tyr Ser Met Val Gly Asn Trp Ala Lys Val Leu Ile Val Met Leu
                365                 370                 375
CTC TTT GCC GGC GTC GAC GGG AGA ACC CAA GTG ACG GGA GCA CAG GCA 1500
Leu Phe Ala Gly Val Asp Gly Arg Thr Gln Val Thr Gly Ala Gln Ala
            380                 385                 390
GGC CAC ACC ACC AGT GGC CTT GCG TCC CTC TTC ACA CCA GGG CCG TCT 1548
Gly His Thr Thr Ser Gly Leu Ala Ser Leu Phe Thr Pro Gly Pro Ser
        395                 400                 405
CAG AAA ATC CAG CTT GTA AAC TCT AAC GGC AGT CGG CAC ATC AAC AGA 1596
Gln Lys Ile Gln Leu Val Asn Ser Asn Gly Ser Arg His Ile Asn Arg
    410                 415                 420
ACT GCC CTG AGT TGC AAC GAC TCC CTC AAA ACT GGC TTC CTT GCC GCG 1644
Thr Ala Leu Ser Cys Asn Asp Ser Leu Lys Thr Gly Phe Leu Ala Ala
425                 430                 435                 440

CTG TTC TAC ACA CAT AAG TTC AAC GCG GCT GGA TGC CCG GAG CGC ATG 1692
Leu Phe Tyr Thr His Lys Phe Asn Ala Ala Gly Cys Pro Glu Arg Met
                445                 450                 455
GCC AGC TGC TGC TCT ATT GAC ACG TTC GAC CAA GGG TGG GGT CCC ATC 1740
Ala Ser Cys Cys Ser Ile Asp Thr Phe Asp Gln Gly Trp Gly Pro Ile
            460                 465                 470
ACC CAT GCT GAG TCT CGC AGC TCG GAC CAA AGG CCT TAC TGC TGG CAC 1788
Thr His Ala Glu Ser Arg Ser Ser Asp Gln Arg Pro Tyr Cys Trp His
        475                 480                 485
TAC GCA CCT CAA CCG TGT GGT ATC GTG CCC GCG TTG CAG GTG TGC GGT 1836
Tyr Ala Pro Gln Pro Cys Gly Ile Val Pro Ala Leu Gln Val Cys Gly
    490                 495                 500
CCA GTG TAT TGT TTC ACC CCA AGC CCT GTT GTA GTG GGG ACG ACC GAC 1884
Pro Val Tyr Cys Phe Thr Pro Ser Pro Val Val Val Gly Thr Thr Asp
505                 510                 515                 520
CGT TTC GGC GTC CCC ACG TAT AAC TGG GGG GAC AAT GAG ACG GAC GTG 1932
Arg Phe Gly Val Pro Thr Tyr Asn Trp Gly Asp Asn Glu Thr Asp Val
                525                 530                 535
CTA CTC CTC AAC AAC ACG CGG CCG CCG CAA GGA AAC TGG TTC GGC TGT 1980
Leu Leu Leu Asn Asn Thr Arg Pro Pro Gln Gly Asn Trp Phe Gly Cys
            540                 545                 550
ACA TGG ATG AAC AGC ACC GGG TTC ACC AAG ACG TGC GGG GGC CCC CCG 2028
Thr Trp Met Asn Ser Thr Gly Phe Thr Lys Thr Cys Gly Gly Pro Pro
        555                 560                 565
TGT AAT ATC GGG GGG GCC GGC AAC AAC ACC TTG ACC TGC CCC ACG GAT 2076
Cys Asn Ile Gly Gly Ala Gly Asn Asn Thr Leu Thr Cys Pro Thr Asp
    570                 575                 580
TGC TTC CGG AAG CAC CCC GAG GCC ACT TAC ACC AAG TGC GGC TCG GGG 2124
Cys Phe Arg Lys His Pro Glu Ala Thr Tyr Thr Lys Cys Gly Ser Gly
585                 590                 595                 600
CCT TGG TTG ACA CCT AGG TGC ATG GTT GAC TAC CCA TAC AGA CTT TGG 2172
Pro Trp Leu Thr Pro Arg Cys Met Val Asp Tyr Pro Tyr Arg Leu Trp
                605                 610                 615
CAC TAC CCC TGC ACT TTC AAC TTT ACC ATC TTT AAG ATC AGG ATG TAT 2220
His Tyr Pro Cys Thr Phe Asn Phe Thr Ile Phe Lys Ile Arg Met Tyr
            620                 625                 630

GTG GGG GGT GTG GAG CAC AGG CTC AAC GCA GCG TGC AAC TGG ACT CGA 2268
Val Gly Gly Val Glu His Arg Leu Asn Ala Ala Cys Asn Trp Thr Arg
        635                 640                 645
GGA GAG CGT TGT AAC ATA GAG GAC AGG GAT AGG TCA GAG CTT AGC CCG 2316
Gly Glu Arg Cys Asn Ile Glu Asp Arg Asp Arg Ser Glu Leu Ser Pro
    650                 655                 660
CTG CTA CTG TCT ACA ACG GAG TGG CAG ATA CTA CCC TGT TCC TTT ACC 2364
Leu Leu Leu Ser Thr Thr Glu Trp Gln Ile Leu Pro Cys Ser Phe Thr
665                 670                 675                 680
ACC CTA CCA GCT CTG TCC ACT GGT TTG ATC CAC CTC CAT CAG AAC ATC 2412
Thr Leu Pro Ala Leu Ser Thr Gly Leu Ile His Leu His Gln Asn Ile
                685                 690                 695
GTG GAC GTG CAA TAC CTG TAC GGT GTA GGG TCA GCA GTT GTC TCC ATT 2460
Val Asp Val Gln Tyr Leu Tyr Gly Val Gly Ser Ala Val Val Ser Ile
            700                 705                 710
GTG ATC AAA TGG GAG TAC GTT CTG CTG CTC TTC CTC CTC CTG GCG GAC 2508
Val Ile Lys Trp Glu Tyr Val Leu Leu Leu Phe Leu Leu Leu Ala Asp
        715                 720                 725
GCG CGC GTC TGC GCC TGC TTA TGG ATG ATG CTG CTG ATA GCC CAG GCT 2556
Ala Arg Val Cys Ala Cys Leu Trp Met Met Leu Leu Ile Ala Gln Ala
    730                 735                 740
GAG GCC ACC TTA GAA AAC CTG GTG GTC CTC AAT TCT GCG TCC GTG GCC 2604
Glu Ala Thr Leu Glu Asn Leu Val Val Leu Asn Ser Ala Ser Val Ala
745                 750                 755                 760
GGA GCG CAT GGC ATC CTC TCA TTT CTT GTG TTC TTC TGT GCA GCC TGG 2652
Gly Ala His Gly Ile Leu Ser Phe Leu Val Phe Phe Cys Ala Ala Trp
                765                 770                 775
TAC ATC AAG GGC AGG CTG GTC CCT GGG GCG GCA TAT GCT TTA TAT GGC 2700
Tyr Ile Lys Gly Arg Leu Val Pro Gly Ala Ala Tyr Ala Leu Tyr Gly
            780                 785                 790
GTG TGG CCG CTG CTC CTG CTC CTG TTG GCG TTA CCA CCA CGG GCA TAC 2748
Val Trp Pro Leu Leu Leu Leu Leu Leu Ala Leu Pro Pro Arg Ala Tyr
        795                 800                 805
GCC ATG GAC CGG GAG ATG GCC GCA TCG TGC GGA GGC GCG GTT TTC GTA 2796
Ala Met Asp Arg Glu Met Ala Ala Ser Cys Gly Gly Ala Val Phe Val
    810                 815                 820

GGT CTA GCA CTC TTG ACC TTG TCA CCA CAC TAT AAA GCA TTC CTC GCC 2844
Leu Thr Leu Ser Gly Leu Ala Leu Pro His Tyr Lys Ala Phe Leu Ala
825                 830                 835                 840
AGG CTC ATA TGG TGG TTA CAA TAT TTT ATC ACC AGG GCT GAG GCG TGC 2892
Arg Leu Ile Trp Trp Leu Gln Tyr Phe Ile Thr Arg Ala Glu Ala Cys
                845                 850                 855
TTG CAA GTG TGG GTC CCC CCT CTC AAC GCA CGG GGG GGC CGC GAT GCC 2940
Leu Gln Val Trp Val Pro Pro Leu Asn Ala Arg Gly Gly Arg Asp Ala
            860                 865                 870
ATC ATC CTC CTC ACG TGC GCG GTC CAC TCA GAG CTA ATC TTT GAC ATC 2988
Ile Ile Leu Leu Thr Cys Ala Val His Ser Glu Leu Ile Phe Asp Ile
        875                 880                 885
ACC AAA ATT CTG CTC GCC ATA CTA GGA CCG CTC ATG GTG CTC CAG GCT 3036
Thr Lys Ile Leu Leu Ala Ile Leu Gly Pro Leu Met Val Leu Gln Ala
    890                 895                 900
GGC ATT ACT AGA GTG CCG TAC TTC GTG CGC GCT CAA GGG CTC ATA CGT 3084
Gly Ile Thr Arg Val Pro Tyr Phe Val Arg Ala Gln Gly Leu Ile Arg
905                 910                 915                 920
GCA TGC ATG TTG GTG CGG AAG GTC GCT GGG GGC CAT TAT GTC CAA ATG 3132
Ala Cys Met Leu Val Arg Lys Val Ala Gly Gly His Tyr Val Gln Met
                925                 930                 935
GCT CTC ATG AAG CTG GCC GCA CTG ACT GGG ACG TAC ATA TAT GAC CAT 3180
Ala Leu Met Lys Leu Ala Ala Leu Thr Gly Thr Tyr Ile Tyr Asp His
            940                 945                 950
CTT ACT CCG CTG CGG GAC TGG GAT CGG TCG GGC CTG CGA GAC CTT GTG 3228
Leu Thr Pro Leu Arg Asp Trp Asp Arg Ser Gly Leu Arg Asp Leu Val
        955                 960                 965
GTA GCG GTC GAG CCC GTC GTC TTC TCC GAC ATG GAG ACC AAG ATC ATC 3276
Val Ala Val Glu Pro Val Val Phe Ser Asp Met Glu Thr Lys Ile Ile
    970                 975                 980
ACC TGG GGG GCG GAC ACA GCG GCG TGT GGG GAC ATC ATC TTG GGT CTG 3324
Thr Trp Gly Ala Asp Thr Ala Ala Cys Gly Asp Ile Ile Leu Gly Leu
985                 990                 995                 1000
CCC GTC TCC GCC CGA AGG GGG AAA GAG ATA CTT CTG GGA CCG GCC GAT 3372
Pro Val Ser Ala Arg Arg Gly Lys Glu Ile Leu Leu Gly Pro Ala Asp
                1005                1010                1015

TCA TTC AGA GAG CAG GGA CTC CGA CTC CTT GCG CCC ATC ACG GCC TAC 3420
Ser Phe Arg Glu Gln Gly Leu Arg Leu Leu Ala Pro Ile Thr Ala Tyr
            1020                1025                1030
TCC CAA CAG ACG CGG GGC CTA TTT GGC TGC ATC GTC ACT AGT CTC ACA 3468
Ser Gln Gln Thr Arg Gly Leu Phe Gly Cys Ile Val Thr Ser Leu Thr
        1035                1040                1045
GGT CGG GAC AAG AAC CAG GTC GAA GGG GAG GCT CAG GTG CTA TCC ACC 3516
Gly Arg Asp Lys Asn Gln Val Glu Gly Glu Ala Gln Val Leu Ser Thr
    1050                1055                1060
GCA ACG CAA TCT TTC TTG GCG ACC TGT GTC AAC GGC GTG TGT TGG ACT 3564
Ala Thr Gln Ser Phe Leu Ala Thr Cys Val Asn Gly Val Cys Trp Thr
1065                1070                1075                1080
GTT TAC CAT GGC GCC GGC TCA AAG ACC CTA GCC GGC CCA AAG GGG CCA 3612
Val Tyr His Gly Ala Gly Ser Lys Thr Leu Ala Gly Pro Lys Gly Pro
                1085                1090                1095
ATC AAC CAA ATG TAC ACT AAT GTG GAT CAG GAC CTC GTC GGC TGG CAG 3660
Ile Asn Gln Met Tyr Thr Asn Val Asp Gln Asp Leu Val Gly Trp Gln
            1100                1105                1110
GCG CCC TCC GGG GCG GCT TCC TTG ACA CCG TGC ACC TAC GGC AGC TCG 3708
Ala Pro Ser Gly Ala Ala Ser Leu Thr Pro Cys Thr Tyr Gly Ser Ser
        1115                1120                1125
GAC CTT TAC TTG GTC ACG AGG CAT GCT GAT GTC ATT CCG GTG CGC CGG 3756
Asp Leu Tyr Leu Val Thr Arg His Ala Asp Val Ile Pro Val Arg Arg
    1130                1135                1140
CGG GGC GAC AGC AGG GGG AGT CTA CTT TCC CCC AGG CCC GTC TCC TAC 3804
Arg Gly Asp Ser Arg Gly Ser Leu Leu Ser Pro Arg Pro Val Ser Tyr
1145                1150                1155                1160
TTG AAG GGC TCT TCC GGT GGT CCA CTG CTC TGC CCC TCG GGG CAC GCT 3852
Leu Lys Gly Ser Ser Gly Gly Pro Leu Leu Cys Pro Ser Gly His Ala
                1165                1170                1175
GTG GGC ATC TTC CGG GCT GCT GTG TGC ACC CGG GGG GTT GCG AAG GCG 3900
Val Gly Ile Phe Arg Ala Ala Val Cys Thr Arg Gly Val Ala Lys Ala
            1180                1185                1190
GTG GAC TTC GTC CCC GTT GAG TCT ATG GAA ACT ACC ATG CGG TCT CCG 3948
Val Asp Phe Val Pro Val Glu Ser Met Glu Thr Thr Met Arg Ser Pro
        1195                1200                1205

GTC TTC ACG GAT AAT TCT TCT CCC CCG GCC GTA CCG CAG ACA TTC CAA 3996
Val Phe Thr Asp Asn Ser Ser Pro Pro Ala Val Pro Gln Thr Phe Gln
    1210                1215                1220
GTG GCC CAT CTA CAT GCT CCC ACT GGC AGC GGC AAG AGC ACT AAG GTG 4044
Val Ala His Leu His Ala Pro Thr Gly Ser Gly Lys Ser Thr Lys Val
1225                1230                1235                1240
CCG GCT GCA TAC GCA GCC CAA GGG TAC AAG GTA CTC GTC CTG AAC CCG 4092
Pro Ala Ala Tyr Ala Ala Gln Gly Tyr Lys Val Leu Val Leu Asn Pro
                1245                1250                1255
TCC GTT GCC GCC ACC TTG GGT TTT GGG GCA TAC ATG TCC AAG GCA CAT 4140
Ser Val Ala Ala Thr Leu Gly Phe Gly Ala Tyr Met Ser Lys Ala His
            1260                1265                1270
GGT GTT GAC CCT AAC ATC AGT ACT GGG GTA AGG ACC ATC ACC ACG GGC 4188
Gly Val Asp Pro Asn Ile Ser Thr Gly Val Arg Thr Ile Thr Thr Gly
        1275                1280                1285
GCC CCT ATC ACG TAC TCC ACC TAC GGC AAG TTC CTT GCC GAC GGT GGT 4236
Ala Pro Ile Thr Tyr Ser Thr Tyr Gly Lys Phe Leu Ala Asp Gly Gly
    1290                1295                1300
TGC TCC GGG GGC GCC TAT GAC ATC ATA ATA TGT GAC GAG TGC CAT TCA 4284
Cys Ser Gly Gly Ala Tyr Asp Ile Ile Ile Cys Asp Glu Cys His Ser
1305                1310                1315                1320
ACT GAC TCG ACT ACA ATC TTA GGC ATC GGC ACG GTC CTG GAC CAA GCG 4332
Thr Asp Ser Thr Thr Ile Leu Gly Ile Gly Thr Val Leu Asp Gln Ala
                1325                1330                1335
GAG ACG GCT GGA GCG CGG CTC GTC GTG CTC GCC GCC GCT ACG CCT CCG 4380
Glu Thr Ala Gly Ala Arg Leu Val Val Leu Ala Ala Ala Thr Pro Pro
            1340                1345                1350
GGA TCG ATA ACC GTG CCA CAT CCC AAC ATT GAG GAG GTG GCC TTG CCT 4428
Gly Ser Ile Thr Val Pro His Pro Asn Ile Glu Glu Val Ala Leu Pro
        1355                1360                1365
AAC ACC GGA GAG ATT CCC TTC TAT GGC AAA GCC ATC CCC ATA GAG ACC 4476
Asn Thr Gly Glu Ile Pro Phe Tyr Gly Lys Ala Ile Pro Ile Glu Thr
    1370                1375                1380
ATC CGC GGG GGG AGG CAT CTC ATC TTC TGC CAT TCC AAG AAG AAG TGT 4524
Ile Arg Gly Gly Arg His Leu Ile Phe Cys His Ser Lys Lys Lys Cys
1385                1390                1395                1400

GAT GAG CTC GCT GCA AAG CTG TCG GGG CTC GGG GTC AAT GCT GTA GCG 4572
Asp Glu Leu Ala Ala Lys Leu Ser Gly Leu Gly Val Asn Ala Val Ala
                1405                1410                1415
TAC TAC CGG GGC CTT GAT GTG TCC GTC ATA CCG ACA ATT GGA GAC GTC 4620
Tyr Tyr Arg Gly Leu Asp Val Ser Val Ile Pro Thr Ile Gly Asp Val
            1420                1425                1430
GTT GTC GTG GCA ACA GAC GCC CTA ATG ACA GGC TTT ACC GGT GAC TTT 4668
Val Val Val Ala Thr Asp Ala Leu Met Thr Gly Phe Thr Gly Asp Phe
        1435                1440                1445
GAT TCA GTG ATC GAC TGT AAT ACA TGT GTC ACC CAG ACA GTC GAC TTC 4716
Asp Ser Val Ile Asp Cys Asn Thr Cys Val Thr Gln Thr Val Asp Phe
    1450                1455                1460
AGC TTG GAC CCT ACC TTC ACC ATT GAG ACG ACA ACG GTC CCC CAA GAC 4764
Ser Leu Asp Pro Thr Phe Thr Ile Glu Thr Thr Thr Val Pro Gln Asp
1465                1470                1475                1480
GCG GTG TCG CGC TCA CAA CGG CGA GGC AGA ACT GGC AGG GGT AGA GGG 4812
Ala Val Ser Arg Ser Gln Arg Arg Gly Arg Thr Gly Arg Gly Arg Gly
                1485                1490                1495
GGC ATA TAC AGG TTT GTG ACT CCA GGG GAA CGG CCT TCG GGT ATG TTC 4860
Gly Ile Tyr Arg Phe Val Thr Pro Gly Glu Arg Pro Ser Gly Met Phe
            1500                1505                1510
GAT TCT TCG GTC CTA TGT GAG TGC TAT GAC GCG GGC TGT GCT TGG TAC 4908
Asp Ser Ser Val Leu Cys Glu Cys Tyr Asp Ala Gly Cys Ala Trp Tyr
        1515                1520                1525
GAG CTC ACG CCC GCA GTG ACC TCG GTT AGG CTG CGG GCT TAC CTA AAT 4956
Glu Leu Thr Pro Ala Val Thr Ser Val Arg Leu Arg Ala Tyr Leu Asn
    1530                1535                1540
ACA CCA GGG TTG CCC GTC TGC CAG GTC CAC CTG GAG TTC TGG GAG AGC 5004
Thr Pro Gly Leu Pro Val Cys Gln Val His Leu Glu Phe Trp Glu Ser
1545                1550                1555                1560
GTC TTT ACA GGC CTC ACC CAC ATA GAT GCC CAC TTC TTG TCC CAG ACT 5052
Val Phe Thr Gly Leu Thr His Ile Asp Ala His Phe Leu Ser Gln Thr
                1565                1570                1575
AAG CAG GCA GGA GAC AAC TTC CCC TAC CTG GTG GCA TAC CAG GCC ACA 5100
Lys Gln Ala Gly Asp Asn Phe Pro Tyr Leu Val Ala Tyr Gln Ala Thr
            1580                1585                1590

GTG TGC GCC AGG GCC CAG GCC CCA CCT CCA TCG TGG GAT CAA ATG TGG 5148
Val Cys Ala Arg Ala Gln Ala Pro Pro Pro Ser Trp Asp Gln Met Trp
        1595                1600                1605
AAG TGT CTC ATA CGG CTA AAG CCT ACG CTG CAC GGG CCA ACA CCC CTG 5196
Lys Cys Leu Ile Arg Leu Lys Pro Thr Leu His Gly Pro Thr Pro Leu
    1610                1615                1620
TTG TAT AGG CTA GGA GCC GTC CAA AAC GAG GTC ACC CTC ACA CAC CCC 5244
Leu Tyr Arg Leu Gly Ala Val Gln Asn Glu Val Thr Leu Thr His Pro
1625                1630                1635                1640
ATA ACC AAA TTC ATC ATG GCA TGC ATG TCG GCT GAT CTA GAG GTC GTC 5292
Ile Thr Lys Phe Ile Met Ala Cys Met Ser Ala Asp Leu Glu Val Val
                1645                1650                1655
ACC AGC ACC TGG GTG CTG GTA GGC GGA GTC CTC GCA GCT CTG GCC GCG 5340
Thr Ser Thr Trp Val Leu Val Gly Gly Val Leu Ala Ala Leu Ala Ala
            1660                1665                1670
TAC TGC CTG ACA ACG GGC AGC GTG GTT ATT GTG GGC AGG ATC ATC TTG 5388
Tyr Cys Leu Thr Thr Gly Ser Val Val Ile Val Gly Arg Ile Ile Leu
        1675                1680                1685
TCC GGG AGG CCG GCT GTG GTA CCT GAC AGG GAA CTG CTC TAC CAG GAG 5436
Ser Gly Arg Pro Ala Val Val Pro Asp Arg Glu Leu Leu Tyr Gln Glu
    1690                1695                1700
TTT GAT GAG ATG GAG GAG TGC GCC TCA CAC CTT CCT TAC ATC GAA CAA 5484
Phe Asp Glu Met Glu Glu Cys Ala Ser His Leu Pro Tyr Ile Glu Gln
1705                1710                1715                1720
GGA ATG CAG CTC GCC GAG CAA TTC AAG CAG AAG GCG CTC GGG CTG CTG 5532
Gly Met Gln Leu Ala Glu Gln Phe Lys Gln Lys Ala Leu Gly Leu Leu
                1725                1730                1735
CAA ACA GCC ACG AAG CAA GCG GAG GCT GCT GCT CCT GTG GTA GAG TCC 5580
Gln Thr Ala Thr Lys Gln Ala Glu Ala Ala Ala Pro Val Val Glu Ser
            1740                1745                1750
AAG TGG AGG GCC CTT GAG GCC TTC TGG GCG AAG CAC ATG TGG AAC TTC 5628
Lys Trp Arg Ala Leu Glu Ala Phe Trp Ala Lys His Met Trp Asn Phe
        1755                1760                1765
ATC AGC GGG ATA CAG TAT CTA GCA GGC TTG TCC ACC CTG CCT GGA AAC 5676
Ile Ser Gly Ile Gln Tyr Leu Ala Gly Leu Ser Thr Leu Pro Gly Asn
    1770                1775                1780

CCC GCA ATA GTA TCA CTG ATG GCA TTC ACA GCC TCT ATC ACT AGC CCG 5724
Pro Ala Ile Val Ser Leu Met Ala Phe Thr Ala Ser Ile Thr Ser Pro
1785                1790                1795                1800
CTC ACC ACC CAA CAT ACC CTC CTG TTT AAC ATC TTA GGG GGA TGG GTG 5772
Leu Thr Thr Gln His Thr Leu Leu Phe Asn Ile Leu Gly Gly Trp Val
                1805                1810                1815
GCC GCC CAA CTC GCC CCC CCC AGC GCT GCT TCA GCC TTC GTG GGC GCC 5820
Ala Ala Gln Leu Ala Pro Pro Ser Ala Ala Ser Ala Phe Val Gly Ala
            1820                1825                1830
GGC ATC GCT GGC GCG GCT GTT GGC AGC ATA GGC CTT GGG AAG GTG CTT 5868
Gly Ile Ala Gly Ala Ala Val Gly Ser Ile Gly Leu Gly Lys Val Leu
        1835                1840                1845
GTG GAC ATC CTG GCG GGT TAT GGA GCA GGG GTG GCA GGC GCA CTC GTG 5916
Val Asp Ile Leu Ala Gly Tyr Gly Ala Gly Val Ala Gly Ala Leu Val
    1850                1855                1860
GCC TTT AAG GGC ATG AGC GGC GAA ATG CCC TCC ACC GAG GAC CTG GTC 5964
Ala Phe Lys Gly Met Ser Gly Glu Met Pro Ser Thr Glu Asp Leu Val
1865                1870                1875                1880
AAC TTA CTC CCT GCC ATC CTC TCT CCT GGC GCC CTG GTC GTC GGG GTC 6012
Asn Leu Leu Pro Ala Ile Leu Ser Pro Gly Ala Leu Val Val Gly Val
                1885                1890                1895
GTG TGC GCA GCA ATA CTG CGT CGG CAT GTG GGC CCA GGG GAG GGG GCT 6060
Val Cys Ala Ala Ile Leu Arg Arg His Val Gly Pro Gly Glu Gly Ala
            1900                1905                1910
GTG CAG TGG ATG AAC CGG CTG ATA GCA TTC GCC TCG CGG GGT AAC CAC 6108
Val Gln Trp Met Asn Arg Leu Ile Ala Phe Ala Ser Arg Gly Asn His
        1915                1920                1925
GTT TCC CCC ACG CAT TAT GTG CCT GAG AGC GAT GCT GCG GCG CGT GTC 6156
Val Ser Pro Thr His Tyr Val Pro Glu Ser Asp Ala Ala Ala Arg Val
    1930                1935                1940
ACC AAG ATC CTC TCC AGC CTT ACC ATC ACT CAG CGT CTA AAA AGG CTC 6204
Thr Lys Ile Leu Ser Ser Leu Thr Ile Thr Gln Arg Leu Lys Arg Leu
1945                1950                1955                1960
CAC CAG TGG ATT AAT GAG GAC TGC TCC ACG CCA TGC TCC GGT TCG TGG 6252
His Gln Trp Ile Asn Glu Asp Cys Ser Thr Pro Cys Ser Gly Ser Trp
                1965                1970                1975

CTC AAA GAT GTT TGG GAC TGG ATA TGC ACG GTG TTG ACT GAC TTC AAG 6300
Leu Lys Asp Val Trp Asp Trp Ile Cys Thr Val Leu Thr Asp Phe Lys
            1980                1985                1990
ACC TGG CTC CAG TCC AAG CTC CTG CCG CGG CTG CCG GGA GAC CCC TTC 6348
Thr Trp Leu Gln Ser Lys Leu Leu Pro Arg Leu Pro Gly Asp Pro Phe
        1995                2000                2005
TTC TCA TGT CAA CGT GGA TAC AGG GGG GTC TGG CGG GGA GAT GGC GTC 6396
Phe Ser Cys Gln Arg Gly Tyr Arg Gly Val Trp Arg Gly Asp Gly Val
    2010                2015                2020
ATG CAA ACC ACC TGC CCA TGT GGA GCA CAG ATC ACC GGA CAT GTC AAA 6444
Met Gln Thr Thr Cys Pro Cys Gly Ala Gln Ile Thr Gly His Val Lys
2025                2030                2035                2040
AAC GGT TCC ATG AGG ATC GTT GGG CCT AAA ACC TGT AGC AAC ACG TGG 6492
Asn Gly Ser Met Arg Ile Val Gly Pro Lys Thr Cys Ser Asn Thr Trp
                2045                2050                2055
CAC GGA ACA TTC CCC ATC AAC GCA TAC ACC ACG GGC CCC TGC ACG CCC 6540
His Gly Thr Phe Pro Ile Asn Ala Tyr Thr Thr Gly Pro Cys Thr Pro
            2060                2065                2070
TCC CCG GCG CCA AAC TAT TCC AGG GCG CTG TGG CGG GTG GCT GCT GAG 6588
Ser Pro Ala Pro Asn Tyr Ser Arg Ala Leu Trp Arg Val Ala Ala Glu
        2075                2080                2085
GAG TAT GTG GAA GTC ACG CGG GTG GGG GAT TTC CAC TAC GTG ACG GGC 6636
Glu Tyr Val Glu Val Thr Arg Val Gly Asp Phe His Tyr Val Thr Gly
    2090                2095                2100
ATG ACC ACT GAC AAC GTA AAA TGC CCA TGC CAG GTT CCA GCC CCC GAA 6684
Met Thr Thr Asp Asn Val Lys Cys Pro Cys Gln Val Pro Ala Pro Glu
2105                2110                2115                2120
TTC TTC UCC GAA GTG GAT GGG GTG CGG TTG CAC AGG TAC GCT CCA GCT 6732
Phe Phe Ser Glu Val Asp Gly Val Arg Leu His Arg Tyr Ala Pro Ala
                2125                2130                2135
TGC AGG CCT CTC CTA CGG GAT GAG GTT ACA TTC CAG GTC GGG CTC AAC 6780
Cys Arg Pro Leu Leu Arg Asp Glu Val Thr Phe Gln Val Gly Leu Asn
            2140                2145                2150
CAA TTC TTA GTT GGG TCA CAG CTC CCG TGT GAG CCC GAA CCG GAT GTA 6828
Gln Phe Leu Val Gly Ser Gln Leu Pro Cys Glu Pro Glu Pro Asp Val
        2155                2160                2165

ACA GTG CTC ACT TCC ATG CTT ACC GAC CCC TCC CAC ATT ACA GCG GAG 6876
Thr Val Leu Thr Ser Met Leu Thr Asp Pro Ser His Ile Thr Ala Glu
    2170                2175                2180
ACG GCT AAG CGT AGG TTG GCC AGA GGG TCT CCC CCC TCC TTG GCC AGC 6924
Thr Ala Lys Arg Arg Leu Ala Arg Gly Ser Pro Pro Ser Leu Ala Ser
2185                2190                2195                2200
TCT TCA GCT AGC CAG TTG TCT GCG CCT TCT TTG AAG GCG ACA TGC ACT 6972
Ser Ser Ala Ser Gln Leu Ser Ala Pro Ser Leu Lys Ala Thr Cys Thr
                2205                2210                2215
ACC CGT CAT GTG TCC CCG GAC GCC GAC CTC ATC GAG GCC AAC CTC CTG 7020
Thr Arg His Val Ser Pro Asp Ala Asp Leu Ile Glu Ala Asn Leu Leu
            2220                2225                2230
TGG CGG CAG GAG ATG GGC GGG AAC ATC ACC CGG GTG GAG TCA GAG AAT 7068
Trp Arg Gln Glu Met Gly Gly Asn Ile Thr Arg Val Glu Ser Glu Asn
        2235                2240                2245
AAG GTA GTA GTA CTA GAC TCT TTT GAA CCG ATA CGG GCG GTC GAG GAT 7116
Lys Val Val Val Leu Asp Ser Phe Glu Pro Ile Arg Ala Val Glu Asp
    2250                2255                2260
GAG AGG GAA GTA TCC GTG GCG GCG GAG ATC CTG CGG AAA CCG AAA AAG 7164
Glu Arg Glu Val Ser Val Ala Ala Glu Ile Leu Arg Lys Pro Lys Lys
2265                2270                2275                2280
TTC CCC CCA GCG ATG CCC ATA TGG GCA CGC CCG AGC TAC AAC CCA CCA 7212
Phe Pro Pro Ala Met Pro Ile Trp Ala Arg Pro Ser Tyr Asn Pro Pro
                2285                2290                2295
CTC CTA GAG TCT TGG AAG GAC CCG GAC TAC GTC CCT CCG GTG GTA CAC 7260
Leu Leu Glu Ser Trp Lys Asp Pro Asp Tyr Val Pro Pro Val Val His
            2300                2305                2310
GGG TGC CCT CTG CCA AGC ACC ATG GCC CCT CCA ATA CCA CCT CCA CGG 7308
Gly Cys Pro Leu Pro Ser Thr Met Ala Pro Pro Ile Pro Pro Pro Arg
        2315                2320                2325
AGG AAG AGG ACG GTT GTC CTG ACA GAA TCC TCG GTG TCT TCT GCC TTG 7356
Arg Lys Arg Thr Val Val Leu Thr Glu Ser Ser Val Ser Ser Ala Leu
    2330                2335                2340
GCT GAG CTT GCC ACA AAG ACC TTC GGC AGC TCC GGA TCG TCA GCC GTT 7404
Ala Glu Leu Ala Thr Lys Thr Phe Gly Ser Ser Gly Ser Ser Ala Val
2345                2350                2355                2360

GAC AGC GGC ACG GCG ACG GCC CCT CCT GAC CAG CCC TCC GAT GAC GGA 7452
Asp Ser Gly Thr Ala Thr Ala Pro Pro Asp Gln Pro Ser Asp Asp Gly
                2365                2370                2375
GAC AGA GGA TCT GAC GAT GAG TCG TAC TCC TCC ATG CCC CCC CTT GAG 7500
Asp Arg Gly Ser Asp Asp Glu Ser Tyr Ser Ser Met Pro Pro Leu Glu
            2380                2385                2390
GGG GAG CCG GGG GAC CCC GAT CTC AGC GAC GGG TCT TGG TCT ACC GTG 7548
Gly Glu Pro Gly Asp Pro Asp Leu Ser Asp Gly Ser Trp Ser Thr Val
        2395                2400                2405
AGC GAG GAG GCT AGT GAG GAC GTC GCT TGC TGC TCG ATG TCT TAC ACG 7596
Ser Glu Glu Ala Ser Glu Asp Val Ala Cys Cys Ser Met Ser Tyr Thr
    2410                2415                2420
TGG ACA GGC GCC CTA ATC ACA CCA TGC GCC GCG GAG GAG AGC AAG CTG 7644
Trp Thr Gly Ala Leu Ile Thr Pro Cys Ala Ala Glu Glu Ser Lys Leu
2425                2430                2435                2440
CCC ATC AAT CCG TTG AGC AAC TCT TTG CTG CGT CAC CAC TCG ATG GTC 7692
Pro Ile Asn Pro Leu Ser Asn Ser Leu Leu Arg His His Ser Met Val
                2445                2450                2455
TAC GCC ACA ACA TCT CGC AGC GCA GGC CTA CGG CAG AAA AAG GTC ACC 7740
Tyr Ala Thr Thr Ser Arg Ser Ala Gly Leu Arg Gln Lys Lys Val Thr
            2460                2465                2470
TTT GAC AGA CTG CAG GTC CCG GAC GAC CAT TAC CGG GAC GTG CTC AAG 7788
Phe Asp Arg Leu Gln Val Pro Asp Asp His Tyr Arg Asp Val Leu Lys
        2475                2480                2485
GAG ATG AAG GCG AAG GCG TCC ACA GTT AAG GCT AAA CTT CTA TCT ATT 7836
Glu Met Lys Ala Lys Ala Ser Thr Val Lys Ala Lys Leu Leu Ser Ile
    2490                2495                2500
GAA GAA GCC TGC AAG CTG ACG CCC CCA CAC TCG GCC AGA TCC AAA TTT 7884
Glu Glu Ala Cys Lys Leu Thr Pro Pro His Ser Ala Arg Ser Lys Phe
2505                2510                2515                2520
GGC TAT GGG GCG AAG GAC GTC CGG AAC CTA TCC AGC AGA GCC GTT AAC 7932
Gly Tyr Gly Ala Lys Asp Val Arg Asn Leu Ser Ser Arg Ala Val Asn
                2525                2530                2535
CAC ATC CAC TCC GTG TGG GAA GAC TTG CTG GAA GAC ACT GAA ACA CCA 7980
His Ile His Ser Val Trp Glu Asp Leu Leu Glu Asp Thr Glu Thr Pro
            2540                2545                2550

ATT GAC ACT ACC ATC ATG GCA AAA AAT GAG GTC TTC TGT GTT CAA CCA 8028
Ile Asp Thr Thr Ile Met Ala Lys Asn Glu Val Phe Cys Val Gln Pro
        2555                2560                2565
GAG AAA GGA GGC CGC AAG CCA GCC CGC CTT ATC GTA TTC CCA GAA CTT 8076
Glu Lys Gly Gly Arg Lys Pro Ala Arg Leu Ile Val Phe Pro Glu Leu
    2570                2575                2580
GGG GTT CGT GTG TGC GAG AAA ATG GCC CTT TAC GAC GTG GTC TCC ACT 8124
Gly Val Arg Val Cys Glu Lys Met Ala Leu Tyr Asp Val Val Ser Thr
2585                2590                2595                2600
CTT CCT CAG GTG GTG ATG GGC CCG TCA TAC GGA TTC CAG TAC TCT CCT 8172
Leu Pro Gln Val Val Met Gly Pro Ser Tyr Gly Phe Gln Tyr Ser Pro
                2605                2610                2615
GGG CAG CGG GTC GAG TTC CTG GTG AAT ACG TGG AAA TCG AAG AAA AAC 8220
Gly Gln Arg Val Glu Phe Leu Val Asn Thr Trp Lys Ser Lys Lys Asn
            2620                2625                2630
CCT ATG GGC TTC GCA TAT TGC ACC CGC TGT TTT GAC TCA ACG GTC ACT 8268
Pro Met Gly Phe Ala Tyr Cys Thr Arg Cys Phe Asp Ser Thr Val Thr
        2635                2640                2645
GAG AGT GAT ATC CGT GTT GAG GAG TCA ATT TAC CAA TGT TGT GAC TTG 8316
Glu Ser Asp Ile Arg Val Glu Glu Ser Ile Tyr Gln Cys Cys Asp Leu
    2650                2655                2660
GCC CCC GAG GCC AGA CAG GTC ATA AGG TCG CTC ACG GAG CGG CTT TAT 8364
Ala Pro Glu Ala Arg Gln Val Ile Arg Ser Leu Thr Glu Arg Leu Tyr
2665                2670                2675                2680
ATC GGG GGC CCC CTG ACT AAT TCA AAA GGG CAG AAC TGC GGT TAT CGC 8412
Ile Gly Gly Pro Leu Thr Asn Ser Lys Gly Gln Asn Cys Gly Tyr Arg
                2685                2690                2695
CGG TGC CGC GCC AGC GGT GTG CTG ACG ACT AAC TGC GGT AAT ACC CTC 8460
Arg Cys Arg Ala Ser Gly Val Leu Thr Thr Asn Cys Gly Asn Thr Leu
            2700                2705                2710
ACA TGT TAC TTG AAG GCC TCT GCA GCC TGT CGA GCT GCA AAG CTC CAG 8508
Thr Cys Tyr Leu Lys Ala Ser Ala Ala Cys Arg Ala Ala Lys Leu Gln
        2715                2720                2725
GAC TGC ACG ATG CTC GTG TGC GGA GAC GAC CTT GTC GTT ATC TGT GAA 8556
Asp Cys Thr Met Leu Val Cys Gly Asp Asp Leu Val Val Ile Cys Glu
    2730                2735                2740

AGC GCG GGA ACC CAG GAG GAC GCG GCG AGC CTA CGA GTC TTC ACG GAG 8604
Ser Ala Gly Thr Gln Glu Asp Ala Ala Ser Leu Arg Val Phe Thr Glu
2745                2750                2755                2760
GCT ATG ACT AGG TAC TCT GCC CCC CCC GGG GAC CCG CCC CAA CCA GAA 8652
Ala Met Thr Arg Tyr Ser Ala Pro Pro Gly Asp Pro Pro Gln Pro Glu
                2765                2770                2775
TAC GAC TTG GAG TTA ATA ACA TCA TGC TCC TCC AAC GTG TCG GTC GCG 8700
Tyr Asp Leu Glu Leu Ile Thr Ser Cys Ser Ser Asn Val Ser Val Ala
            2780                2785                2790
CAC GAC GCA TCT GGC AAG CGG GTG TAC TAC CTC ACT CGC GAC CCC ACC 8748
His Asp Ala Ser Gly Lys Arg Val Tyr Tyr Leu Thr Arg Asp Pro Thr
        2795                2800                2805
ACC CCC CTC GCG AGG GCA GCG TGG GAA ACA GTT AGA CAC ACT CCA GTA 8796
Thr Pro Leu Ala Arg Ala Ala Trp Glu Thr Val Arg His Thr Pro Val
    2810                2815                2820
AAC TCC TGG CTA GGC AAC ATC ATC ATG TAC GCG CCC ACC CTG TGG GCA 8844
Asn Ser Trp Leu Gly Asn Ile Ile Met Tyr Ala Pro Thr Leu Trp Ala
2825                2830                2835                2840
AGG ATG ATT CTG ATG ACC CAC TTC TTC TCC ATC CTT CTA GCT CAG GAG 8892
Arg Met Ile Leu Met Thr His Phe Phe Ser Ile Leu Leu Ala Gln Glu
                2845                2850                2855
CAA CTT GAA AAA GCC CTG GGT TGT CAG ATC TAC GGG GCC ACT TAC TTC 8940
Gln Leu Glu Lys Ala Leu Gly Cys Gln Ile Tyr Gly Ala Thr Tyr Phe
            2860                2865                2870
ATT GAA CCA CTT GAC CTA CCT CAG ATC ATT GAA CGA CTC CAC GGT CTT 8988
Ile Glu Pro Leu Asp Leu Pro Gln Ile Ile Glu Arg Leu His Gly Leu
        2875                2880                2885
AGC GCA TTT TCA CTC CAC AGT TAC TCT CCA GGT GAA ATC AAT AGG GTG 9036
Ser Ala Phe Ser Leu His Ser Tyr Ser Pro Gly Glu Ile Asn Arg Val
    2890                2895                2900
GCT TCA TGC CTC AGG AAA CTT GGG GTA CCA CCC TTG CGA GTC TGG AGA 9084
Ala Ser Cys Leu Arg Lys Leu Gly Val Pro Pro Leu Arg Val Trp Arg
2905                2910                2915                2920
CAT CGG GCC AGA AGT GTC CGC GCT AAG CTA CTG TCC CAG GGG GGG AGG 9132
His Arg Ala Arg Ser Val Arg Ala Lys Leu Leu Ser Gln Gly Gly Arg
                2925                2930                2935

GCC GCC ACT TGT GGC AAG TAC CTC TTC AAC TGG GCA GTG AAA ACC AAG 9180
Ala Ala Thr Cys Gly Lys Tyr Leu Phe Asn Trp Ala Val Lys Thr Lys
            2940                2945                2950
CTT AAA CTC ACT CCA ATT CCG GCT GCG TCC AGA TTG GAC TTA TCC GGC 9228
Leu Lys Leu Thr Pro Ile Pro Ala Ala Ser Arg Leu Asp Leu Ser Gly
        2955                2960                2965
TGG TTC GTT GCT GGT TAC AAT GGG GGA GAC ATA TAT CAC AGC CTG TCT 9276
Trp Phe Val Ala Gly Tyr Asn Gly Gly Asp Ile Tyr His Ser Leu Ser
    2970                2975                2980
CGT GCC CGA CCC CGC TGG TTC ATG TGG TGC CTA CTC CTA CTT TCT GTA 9324
Arg Ala Arg Pro Arg Trp Phe Met Trp Cys Leu Leu Leu Leu Ser Val
2985                2990                2995                3000
GGG GTA GGC ATC TAC CTG CTC CCC AAC CGG TGA ACGGGGAGCT 9370
Gly Val Gly Ile Tyr Leu Leu Pro Asn Arg ***
                3005                3010
AACCACTCCA GGCCAATAGG CCATTCCCTT TTTTT 9405

## Claims

1. A single-stranded cDNA comprising about 9500 nucleotides and encoding human hepatitis C virus gene, or a double-stranded DNA comprising said single-stranded cDNA and its complimentary single-stranded DNA.

2. The single- or double-stranded cDNA as claimed in Claim 1, wherein said single-stranded cDNA comprises a nucleotide sequence as shown in a Sequence Listing (SEQ ID NO. 1).

3. A method of detection for human C hepatitis virus or diagnosis of human C hepatitis, wherein an RNA derived from a serum sample of an individual and parts of the cDNA as claimed in Claim 1, as primers are subjected to a polymerase chain reaction and then to an electrophoresis.

4. A cDNA clone encoding an amino acid sequence shown in a Sequence Listing (SEQ ID NO. 1).

5. A method of detection for human anti-hepatitis C virus antibody or diagnosis of human hepatitis C, wherein a peptide encoding a part of amino acid sequence shown in a Sequence Listing (SEQ ID NO. 1) is employed as an antigen.

6. A method of production of vaccine for human hepatitis C, wherein a peptide encoding a part of the amino acid sequence shown in the Sequence Listing (SEQ ID NO. 1) is employed as an antigen and attenuated by a method of subculture.
